Europäisches Patentamt

(19) European Patent Office

Office européen des brevets

(11) Publication number : 0 686 623 A1

(12) **EUROPEAN PATENT APPLICATION**

(21) Application number : 95303848.6

(22) Date of filing : 06.06.95

(51) Int. Cl.⁶ : **C07C 69/54,** C07C 67/20, C07C 67/327, C07C 67/58, C07C 231/06, C07C 231/24

(30) Priority : 06.06.94 JP 123408/94

(43) Date of publication of application :
13.12.95 Bulletin 95/50

(84) Designated Contracting States :
DE FR GB IT

(71) Applicant : MITSUI TOATSU CHEMICALS, Inc.
2-5 Kasumigaseki 3-chome
Chiyoda-Ku Tokyo 100 (JP)

(72) Inventor : Inomata, Masamitsu
Miyanodai Dai 2 Apt. 44, 2142 Tougou
Mobara-shi, Chiba (JP)
Inventor : Nomura, Masatoshi
Miyanodai Ryou, 2142 Tougou
Mobara-shi, Chiba (JP)

Inventor : Takeno, Masahiro
Miyanodai Ryou, 2142 Tougou
Mobara-shi, Chiba (JP)
Inventor : Hiraiwa, Takeshi
Nakanodai Shataku 14, 226-1 Takashi
Mobara-shi, Chiba (JP)
Inventor : Tokunoh, Shinji
Miyanodai Ryou, 2142 Tougou
Mobara-shi, Chiba (JP)
Inventor : Nakajima, Yoshiyuki
Miyanodai Ryou, 2142 Tougou
Mobara-shi, Chiba (JP)

(74) Representative : Nicholls, Kathryn Margaret et al
MEWBURN ELLIS
York House
23 Kingsway
London WC2B 6HP (GB)

(54) **Process for producing methyl methacrylate**

(57) There is provided a process for producing MMA characterized by comprising the steps of : introducing an α-hydroxyisobutyric acid amide (HAM) production solution 5 into a thermal decomposition reactor 6 ; decomposing and separating non-reacted acetone cyanohydrin (ACH) to produce a HAM aqueous solution 11 ; contacting the HAM aqueous solution with ion exchange resins 12 and 13 ; dehydrating-hydrating it at a dehydration-hydration reactor 17 to produce methacrylic acid and/or methacrylamide 18 to which methanol 21 is added, which is esterified at an esterification reactor 22 to produce methyl methacrylate (MMA) ; contacting the MMA product 23 with a hydrophobic organic solvent 25 to extract MMA ; separating MMA 42 by distillation ; using the solvent 25, from which MMA is separated, for the liquid-liquid extraction ; recovering methanol and by-product ammonia contained in the raffinate solution 27 ; using the methanol 37 as the starting material for the esterification reaction ; using, for the ACH synthesis, a mixture 10 of the acetone and the hydrocyanic acid discharged from the thermal decomposition reactor 6 ; and recycling the resultant ACH as a starting material for the hydration reaction.

The present invention permits production of MMA of high purity, elongating the lifetime of the catalyst. In addition, the recovered product(s) may be recycled. The present invention is thus suitable for industrial production of MMA.

Fig. 1

EP 0 686 623 A1

The present invention relates to a novel process for producing methyl methacrylate (MMA), in particular, for producing it continuously. More particularly, the present invention relates to a process which may continuously produce MMA of high purity in a stable manner for a long time producing neither a by-product of ammonium sulfate nor a waste acid, by means of liquid phase amidation, dehydration-hydration reaction, and subsequent esterification by using acetone cyanohydrin (ACH), water and methanol used as major starting materials.

Typical processes for the industrial production of MMA are classified into two groups: direct oxidation process using $C_4$ fraction such as isobutylene as a starting material ($C_4$ method) and the ACH method using hydrocyanic acid and acetone as starting materials. The $C_4$ method has been used in practice these days because of the cost-effectiveness of the materials. There still remain problems of, for example, short of materials. As a result, the ACH method has been overwhelmingly the major process for producing MMA in the world.

The conventional ACH method is as follows. ACH is synthesized from hydrocyanic acid and acetone. The resultant ACH is amidated in the presence of excessive sulfuric acid, and is then reacted with methanol to produce MMA. This process has found wide applications because of its capability of providing a high yield through a simple reaction. But disadvantages lie in the corrosion of materials of reaction equipment by the concentrated sulfuric acid used and in a lot of invaluable ammonium sulfate by-product. With this respect, MMA is also produced through a process with sulfuric acid recovery comprising the steps of burning the waste mixture of sulfuric acid and ammonium hydrogen sulfate which are precursors of ammonium sulfate, to recover the sulfuric acid from the burning gas.

However, the above-mentioned process involving in the recovery of the sulfuric acid has various problems including a large equipment cost. Though the sulfuric acid can be recovered, ammonia is converted into nitrogen, which cannot be recovered. In addition, recent environmental policy requires reduction of discharged nitrogen oxides or sulfur oxides. Another problem is that the sulfuric acid corrodes facilities themselves to produce MMA as well as other facilities for recovering the sulfuric acid.

In contrast, a process for producing MMA from ACH without using sulfuric acid is disclosed in, for example, British Patent No. 1,351,530 and U.S. Patent No. 4,018,829. In these processes, ACH and water are supplied to and hydrated in a fixed-bed reactor filled with a manganese dioxide catalyst to produce α-hydroxyisobutyric acid amide (HAM). Next, as is disclosed in Japanese Patent Publication No. 63-63537 or Japanese Patent Publication No. 63-10940 (U.S. Patent 4464539), HAM contacts with a solid acid catalyst in the first stage in the presence of water to produce a reaction product containing methacrylic acid (MAA) and/or methacrylamide (MAM). Subsequently, the reaction product is exposed to a solid acid catalyst in the second stage in the presence of methanol to produce MMA.

The conventional processes disclosed in the above mentioned references have many advantages over preceding process using the sulfuric acid. However, even these processes are not sufficient in view of continuously producing MMA of high purity for a long time and are thus not established as an industrial technology well available.

More specifically, a HAM production solution which resulted from hydrated reaction of ACH typically contains at least a few percents of non-reacted ACH. If this non-reacted ACH is not separated or removed, or if the separation is insufficient, toxic ACH and hydrocyanic acid remain in the reaction solution in the subsequent steps. It is thus very dangerous by the safety considerations.

If hydration of ACH is completed at the conversion rate of 100%, subsequent step of recovering non-reacted ACH is not required. Considering the productivity of a reactor, the industrial production is in operation at a medium conversion rate to achieve the highest productivity of the reactor. In this event, it is important how to recover non-reacted ACH. The first challenge is, therefore, to remove or to separate and to recover the non-reacted ACH from the HAM production solution for the industrial continuous production of MMA from ACH via the intermediate compound, HAM.

The inventors of the present invention produced HAM by means of hydrating ACH until its conversion rate reached to 100% by using a mixed solvent of acetone and water in the presence of a manganese dioxide catalyst in the liquid phase by batch operation, evaporating acetone in HAM production solution, and concentrating the HAM solution to crystallize HAM. With this HAM, long-time synthesis of MAA and/or MAM had been attempted in the presence of water by using a solid-acid catalyst through the vapor-phase reaction. As a result, other problems occur that the conversion rate of HAM was decreased rapidly with time and resulted in decreasing the yield of MAA and/or MAM.

The inventors of the present invention had analyzed the deactivated solid-acid catalyst to find possible causes of the above mentioned problems. The analytical results provided a finding that a large amount of metal species and sulfur therein deactivated the catalyst. More specifically, these components contained in HAM would be the cause of the catalyst deactivation. No way is known for purification of a HAM product obtained as a result of hydration reaction of ACH. In addition, a relation between the purity of HAM and the lifetime of the catalyst for MAA and/or MAM synthesis is unknown. The second challenge is, therefore, to remove cationic and anionic impurities in HAM to maintain the activity of the catalyst for MAA and/or MAM synthesis for a long time.

The process for producing MMA from HAM is well-known and is disclosed in, for example, Japanese Patent Publication Nos. 63-63537 and 63-10940 (U.S. Patent 4464539). These publications describe nothing about how to remove and recover by-product ammonia contained in the resultant product. They also do not mention how to obtain purified MMA from the product after esterification. The third challenge is, therefore, to establish a purification process operable after esterification with an effective method of recovering by-product ammonia.

As mentioned above, the process for producing MMA without using sulfuric acid has no problems of by-production of ammonium sulfate, corrosion of the equipment and pollution, which were inherent to conventional processes such as the ACH method. However, as mentioned above, these processes have not reached to a practical level by the industrial considerations to continuously obtain highly pure MMA for a long time.

The present invention is directed to overcome the above mentioned problems and provides a novel process for producing MMA. More particularly, an object of the present invention is to provide a series of industrial processes for producing MMA which permit continuous production of MMA of high purity for a long time and permit recovery, for recycling, of by-product ammonia produced in a reaction to produce MMA.

The present inventors have made tremendous studies and examinations to achieve the above mentioned object. As a result, following findings were obtained and the present invention was thus completed. (1) It is effective to introduce the HAM production solution obtained by hydration of ACH into a thermal decomposition reactor, heat treat the content therein at a high temperature, decompose any non-reacted ACH in the production solution into acetone and hydrocyanic acid and separate them. ACH can be obtained from these resultant acetone and hydrocyanic acid for recycle to the process of hydration as a starting material. (2) The HAM aqueous solution discharged from the thermal decomposition reactor contains, as mentioned above, a large amount of components that deactivate the catalyst used for MAA and/or MAM synthesis in a subsequent process, and an ion exchange treatment of the HAM solution is useful for removal of such components. (3) To obtain MMA of high purity, effective processes include liquefaction through quenching the MMA product obtained by esterification; to extract MMA from that product in contact with hydrophobic organic solvent; and to separate MMA by distillation of the extract solution. (4) This method is applied to recover readily the by-product ammonia and the non-reacted methanol, which can be recycled advantageously as starting materials.

More specifically, the present invention provides a process for producing MMA characterized by comprising the steps of: introducing a HAM production solution obtained by hydration of ACH into a thermal decomposition reactor; decomposing and separating non-reacted ACH in the solution into acetone and hydrocyanic acid to produce a HAM aqueous solution; contacting the HAM aqueous solution with an ion exchange resin; dehydrating-hydrating it to produce MAA and/or MAM; contacting the MMA product with a hydrophobic organic solvent to extract MMA by liquid-liquid extraction; separating MMA by distillation; recycling the solvent, from which MMA was separated, for the liquid-liquid extraction; recovering methanol and by-product ammonia contained in the raffinate solution by distillation for separation thereof; using the methanol as the starting material for the esterification reaction and the ammonia as the starting material for the hydrocyanic acid; using, for the ACH synthesis, the acetone and the hydrocyanic acid discharged from the thermal decomposition reactor; and recycling the resultant ACH as the starting material for the hydration reaction.

The process according to the present invention provides MMA containing less or no impurities without causing the problems such as corrosion of the equipment, by-production of ammonium sulfate, and waste acid disposal, which are inherent to the conventional ACH methods. Moreover, the process according to the present invention enables to prolong the lifetime of the catalyst used for the dehydration-hydration reaction and esterification reaction. Therefore, this is a preferable process for industrial continuous production, and the realization of which is very significant.

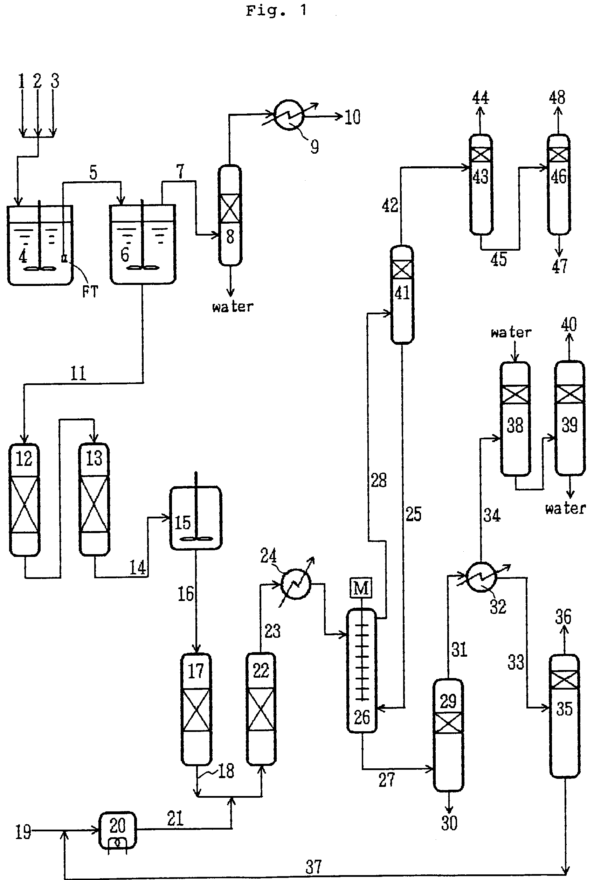

Embodiments of the invention are described below, by way of example only, and with reference to the accompanying single figure, Fig 1.

Fig. 1 is a schematic view for use in describing specifically some steps of a process for producing MMA according to the present invention.

1. ACH
2. water
3. acetone
4. hydration reactor
FT. filter
5. HAM production solution
6. thermal decomposition reactor
7. mixed gas consisting of acetone, hydrocyanic acid and water

8. distillation column

9. condenser

10. mixed solution of acetone and hydrocyanic acid

11. HAM aqueous solution

12. cation exchange resin column

13. anion exchange resin column

14. decationized and deanionized HAM aqueous solution

15. concentration adjusting vessel

16. HAM aqueous solution after adjusting concentration

17. dehydration-hydration reactor

18. dehydration-hydration reaction product

19. methanol

20. methanol evaporator

21. vaporized methanol

22. esterification reactor

23. MMA product

24. quench tower

25. extractant

26. extraction column

27. raffinate solution

28. extract solution

29. methanol recovery column

30. bottom solution of methanol recovery column

31. top gas of methanol recovery column

32. condenser

33. methanol solution

34. ammonia gas

35. recovery column of low boiling point fraction from raffinate solution

36. low boiling point materials

37. recovered methanol

38. ammonia absorption column

39. ammonia purification column

40. purified ammonia

41. extractant recovery column

42. top liquid of extractant recovery column

43. separation column of low boiling point fraction from extract solution

44. low boiling point materials

45. bottom liquid of separation column of low boiling point fraction from extract solution

46. MMA purification column

47. high boiling point materials

48. MMA product

ACH, one of the starting materials used in the present invention, may be obtained from a well-known typical method. A well-known ACH production process is, for instance, to react hydrocyanic acid with acetone under a small amount of alkali or amine as catalysts.

Typically, manganese oxide, whether an anhydride or a hydrate, is used as a catalyst for a hydration reaction of ACH. The manganese oxide may be used in the form of manganese dioxide obtained by means of a well-known technique such as treating potassium permanganate and manganese sulfate under an acidic condition ( Biochem. J., 50 p.43, (1951) and J. Chem. Soc., 1953, p. 2189, (1953) ) and electrolytic oxidation of manganese sulfate aqueous solution. Generally, the catalyst used is in the form of powder having an adequate particle size.

Water, which is a starting material for the hydration reaction of ACH, is also used as a solvent. The amount of water may generally be 1 or more moles, preferably 2-20 moles, and more preferably 4-10 moles relative to 1 mole of ACH. Also, a solvent which is inactive to the reaction may be used along with water as the reaction solvent. For instance, acetone may be used advantageously as disclosed in Japanese Patent Laid Open No. 52-222 (corresponding to U.S. Patent No. 4,018,829 ). The amount of acetone may generally be in the range of from 0.1 to 6.0 moles, preferably from 0.1 to 2.0 mole relative to 1 mole of ACH.

The temperature of the hydration reaction may generally be in the range from 10° to 150°C, preferably from 20° to 100°C, and more preferably from 30° to 80°C. The activity of the catalyst is low at a temperature of lower than 10°C, which is not practical. A temperature higher than 150°C provides a high activity of the catalyst with unpreferably rapidly reducing the yield of HAM.

A fixed-bed catalyst reactor or a suspension-bed catalyst reactor is typically applied for the hydration reaction of ACH. The fixed-bed reactor is a cylindrical vessel filled with a catalyst which is formed into a proper size and shape. In the case of using the suspension-bed reactor, the concentration of a catalyst in a suspension solution may be, but not limited to, 2% or more by weight, and preferably from 5% to 50% by weight. The supply rate of the starting material liquid to the suspension-bed reactor may be from 0.05 to 1.0 parts by weight of ACH per 1 part by weight of the catalyst per 1 hour (WHSV). This suspension-bed reactor should have a filter made of, for example, a metal or glass at the outlet for the HAM production solution not to discharge particles having a smaller size of the suspension catalyst from the reactor. Two or three reactors may be connected in series to conduct the reaction for a higher conversion rate of ACH.

The HAM production solution obtained as a result of the hydration reaction of ACH contains non-reacted ACH, acetone of the reaction solvent, and small amount of hydrocyanic acid. In a conventional system, such HAM production solution was treated under 200-760 Torr in top pressure of the column at a temperature ranging from 20° to 57°C in the distillation column to remove and to recover any low boiling point material such as acetone and hydrocyanic acid therein, and the HAM aqueous solution was obtained as a bottom solution of the distillation column. However, this distillation operation did not permit decomposition of a non-reacted ACH in the HAM production solution, and most portions of ACH remained in the HAM aqueous solution were discharged through the bottom outlet. With this respect, the process according to the present invention is provided with an additional step of a thermal decomposition reaction to decompose the non-reacted ACH contained in the HAM production solution into acetone and hydrocyanic acid.

The step of the thermal decomposition reaction applicable to the present invention is to decompose the non-reacted ACH into acetone and hydrocyanic acid for recovery. A system for this step comprises a thermal decomposition reactor to decompose ACH by heat and an associated distillation column to distill and separate acetone, hydrocyanic acid, and water produced by decomposition. The reactor and the distillation column may be provided either separately or integrally. Considering efficiencies of the apparatus used, it is preferable in the present invention that the HAM production solution is directly introduced into the thermal decomposition reactor and to conduct separation of acetone simultaneously with decomposition and separation of the non-reacted ACH.

The thermal decomposition may be conducted while stirring the content of the reactor to homogenize it. In this event, the reaction system may be kept at a normal pressure or preferably at a reduced pressure to separate immediately acetone and hydrocyanic acid produced by the thermal decomposition. The operating condition by the thermal decomposition reaction may be at a temperature of from 80° to 100°C under a pressure of from 100 to 760 Torr. It takes a long time to decompose and separate ACH at a temperature of lower than 80°C and under a low pressure of around 100 Torr. Moreover, it takes a short time to decompose ACH and separate the products at a temperature of higher than 100°C. Accordingly, there is no problem by the operational considerations. However, this operation is not preferable from the point of thermal efficiency because of an increased amount of water to be distilled and subjected to reflux. Though depending on the temperature and the pressure, a residence time of the solution needed for the thermal decomposition reaction ranges generally from 0.5 to 30 hour, preferably from 1 to 20 hours, and more preferably from 2 to 12 hours. The residence time shorter than 0.5 hours may be insufficient to decompose the non-reacted ACH. On the contrary, the residence time longer than 30 hours provides no further remarkable effect to the increase of time. The acetone and the hydrocyanic acid obtained by the thermal decomposition reaction may be recycled for the production of ACH which is a starting materials for HAM production.

The HAM aqueous solution discharged from the thermal decomposition reactor contains cations and anions such as alkali metal(s), manganese, and sulfuric acid. The alkali metal ion(s) and the sulfuric acid are contained in the starting material, ACH, and it is expected that the manganese ions result from the change and the deterioration of the manganese oxide catalyst. These ions may deactivate the catalyst in the subsequent step of synthesizing MAA and/or MAM. In a method of removing sulfuric acid before the hydration reaction (as disclosed in Japanese Patent Laid Open No. 2-196763), ACH may be unstable and liable to decompose before the hydration reaction. In addition the activity of manganese oxide catalyst is equal or less than the case that the sulfuric acid is not removed, so that this method seems not to be effective. Therefore, instead of the treatment of ACH, the method of the ion exchange treatment of the HAM aqueous solution is very effective, like the method of the present invention.

The process according to the present invention in which the HAM aqueous solution contacts with the ion exchange resin is operated to pass the HAM aqueous solution through a cation exchange resin layer and an anion exchange resin layer. An acidic cation exchange resin or a chelate cation exchange resin is used as the ion exchange resin to remove the cation in the HAM aqueous solution. Examples of the acidic cation exchange resins include strong acidic cation exchange resins obtained by sulfonation of, for example, a styrene-divinylbenzene copolymer and weak acidic cation exchange resins such as methacrylate-divinylbenzene copolymers and acrylate-divinylbenzene copolymers. Examples of the chelate resin include chelate resins of a styrenedivinylbenzene copolymer into which imino-diacetic acid or ethylene amines such as ethylenediamine and ethylenetriamine are introduced, and chelate resins of ethyleneimine.

The basic anion exchange resins are used as ion exchange resins to remove the anions. Examples thereof include strong basic anion exchange resins such as a quaternary ammonium styrene-divinylbenzene copolymer and weak basic anion exchange resins of acid amide in which acrylate-divinylbenzene copolymer is reacted with dimethylaminoalkyl amine.

The columns are filled with the acidic or chelate cation exchange resin and the anion exchange resin, and the HAM aqueous solution is passed therethrough. The solution is flown at a rate not to cause channelling, and the flow rate expressed as a space velocity (SV) which is the volume flow per one hour divided by the volume of the ion exchange resin is preferably from 2 to 60 $hr^{-1}$. The concentration of the HAM aqueous solution is 60% by weight (water/HAM = 3.8 molar ratio) or lower, and preferably from 10% to 40% by weight (water/HAM = 8.6 to 50 molar ratio). The concentration higher than 60% by weight requires frequent regeneration of the ion exchange resin, which is not preferable. The temperature of the solution in the ion exchange resin column is typically set up in a range of from 0° to 80°C. There is no specific limitation on the order of removing the ions from the HAM aqueous solution.

The ion exchanged HAM aqueous solution is concentrated or diluted with water if necessary to adjust a proper concentration and then is transferred to the next process.

Next, as is disclosed in Japanese Patent Publication Nos. 63-63537 and No. 63-10940 (U.S. Patent 4464539), the HAM aqueous solution obtained from aforementioned process contacts with the first step solid acid catalyst for dehydration-hydration reaction and results in the synthesis MAA and/or MAM. Moreover, methanol is added, then it contacts with the second step solid acid catalyst for esterification to produce MMA. Examples of the solid acid catalysts in this reaction include, phosphate such as lanthanum phosphate and cerium phosphate as the catalyst for the first stage, and phosphate and oxide of titanium or zirconium as the catalyst for the second stage.

The amounts of methanol and water used for the above mentioned reaction are not specifically limited. However, the amount of water is generally in a range of from 1 to 50 moles, and preferably from 1 to 15 moles relative to 1 mole of HAM. The amount of methanol is generally in a range of from 1 to 50 moles, and preferably from 3 to 15 moles relative to 1 mole of HAM. With the amount of water smaller than 1 mole relative to 1 mole of HAM, the yield of MAA and/or MAM that are/is the dehydration-hydration reaction product(s) is small. On the contrary, the amount larger than 50 moles results in an excessively large load in the subsequent methanol recovery step with increasing the amount of discharged water. Both cases are thus not preferable.

With the amount of methanol smaller than 1 mole relative to 1 mole of HAM, the yield of MMA that is the esterification reaction product is small. On the contrary, the amount of larger than 50 moles results in an excessively large load in the subsequent methanol recovery step. Both cases are thus not preferable.

As long as the reactants can be contacted with the solid acid catalysts, these reactions may be carried out either in the gas phase or in the liquid phase, preferably in the gas phase or the gas-liquid mixed phase. And any types of reactors such as a fixed bed reactor and a fluidized reactor are applicable.

In the above-mentioned reaction, the reaction temperature, the reaction pressure, and the liquid space velocity (LHSV) of the contact reaction, which may be the same or different between the first and second solid acid catalysts, are generally in the following ranges: the reaction temperature is from 150° to 500°C, more preferably from 200° to 450°C; the reaction pressure may typically be an atmospheric pressure, but an increased or reduced pressure may equally be used. The supply rate of materials, that is, the supply rate of HAM, and methanol may be changed in a wide range depending on the catalysts used, and the reaction temperature etc. A typical supply rate may be, for example, in the range of from 0.05 to 10 $hr^{-1}$ by LHSV. An inactive gas such as nitrogen gas may be accompanied with the reaction materials for the reaction. Moreover, ammonia and aqueous ammonia may be supplied to the catalyst layer for the pre-treatment against the catalyst before initiation of the reaction.

When the reaction proceeds from the first step of the dehydration-hydration reaction to the second step of the esterification, the reaction product of dehydration-hydration reaction is preferably supplied directly into the second step of the esterification reactor without cooling to prevent MAA polymerization. When methanol is added for the esterification reaction, it is preferable that the methanol gas which was previously vaporized is supplied and added to the reaction product of the dehydration-hydration reaction.

The MMA product obtained in the above-mentioned reaction is quenched from a hot gas to the state suitable for the extraction feed used in the subsequent process of extracting MMA, i.e., to the liquid state of from 10° to 70°C. It is important that this cooling is conducted immediately. The reason is that a longer residence time of the MMA product in the form of the hot gas may cause reactions of by-producing MAM from MMA and ammonia therein, and of polymerizing MMA and MAM. These reactions may result in a reduced yield of MMA, and clogging of pipes and other equipment. Upon cooling, water and brain may be used as a cooling medium and a multi-tube heat exchanger may be used as the device.

The MMA product so cooled may be, though varied depending on the amounts of water and methanol used for the above mentioned reaction, a solution comprising from 20% to 70% by weight of methanol, 5% or more by weight of MMA, and 10% or more by weight of water.

In the process according to the present invention, the MMA product so obtained is in the form of a solution having a high methanol concentration. Accordingly, a specific hydrophobic organic solvent is used as an extractant for use in extracting MMA from the MMA product.

The reasons are as follows. A large amount of water is required to extract methanol of high concentration in the MMA extraction process using water as the extractant as in the conventional ACH method with necessitating much heat to recover methanol and increasing the amount of discharged water waste. The methanol concentration may be usually lower than 25% by weight or so in the MMA product obtained according to the conventional ACH method.

In the present invention, the cooled MMA product is supplied to the extraction column and is contacted with the hydrophobic organic solvent to extract MMA contained in the product.

In the present invention, the hydrophobic organic solvent used as the extractant may be, for example, saturated aliphatic hydrocarbons having a boiling point of from 105° to 300°C, saturated aliphatic hydrocarbons having a boiling point of from 60° to 95°C and other solvents, considering the boiling point (100°C) of MMA, the extractability of MMA, the solubility of methanol, and the quantity of heat consumed in the subsequent extractant recovery column. Of these, examples of the solvent include, preferably saturated aliphatic hydrocarbons having a boiling point of from 105° to 300°C, and more preferably saturated aliphatic hydrocarbons having a boiling point of from 120° to 250°C. These hydrocarbons may be used alone, or two or more of them may be mixed with each other within each group. Specific examples of the organic solvent include saturated aliphatic hydrocarbons such as n-hexane (boiling point: 69°C), n-octane (boiling point: 126°C), n-nonane (boiling point: 150°C), n-decane (boiling point: 174°C), n-undecane (boiling point: 194°C), n-dodecane (boiling point: 216°C), n-tridecane (boiling point: 234°C), n-tetradecane (boiling point: 254°C), n-pentadecane (boiling point: 270°C), n-hexadecane (boiling point: 286°C) and isomers thereof; saturated cyclic hydrocarbons such as cyclohexane and methyl cyclohexane; aromatic hydrocarbons such as xylene and toluene; and acetic esters such as butyl acetate and isobutyl acetate. A mixed solvent of the aliphatic saturated hydrocarbons commercially available may also be used such as Shellsol 71 (trade name; available from Shell Japan Co.; fraction with branched chain hydrocarbons having an initial boiling point of 179°C and an end point of 212°C).

The amount of the extractant used for this process may be selected depending on the concentrations of MMA, methanol and water contained in the MMA product. The amount may generally be in the range of from 0.1 to 5 parts by weight relative to 1 part by weight of MMA product.

Moreover, if separation between the extract and the raffinate is not good and if the extractability of MMA is not sufficient enough, the addition of water to the MMA product can improve these separation and the extractability. In this event, water may be added up to the level that the total amount of water used for the above mentioned dehydration-hydration reaction and the additional water becomes 15 or smaller by molar ratio of water/HAM.

A single extraction may often be insufficient for the satisfactory level of the extractability. With this respect, a multiple-extraction or counter-current multi-stage extraction may advantageously be used. In addition, any one of adequate device such as a mixer settler extractor or a continuous counter-current extractor may be used.

The extract after extraction of MMA is then supplied to the extractant recovery column to separate MMA and the extractant through distillation. Polymerization of MMA can be avoided in the addition of the polymerization inhibitor chosen properly in this distillation-separation process. The extractant can be recycled to the extraction column and used again. The boiling point of MMA at a normal pressure is 100°C and in this extractant recovery column, the MMA fraction is obtained from the column bottom if the boiling point of the extractant is lower than that of MMA, while the MMA fraction is obtained from the column top if the boiling point of the extractant is higher than that of MMA.

The raffinate solution of the above-mentioned extracting operation contains methanol, MAA (ammonium salt), MAM, by-product ammonia, and water. The solution may be supplied to the methanol recovery column and distilled therein to separate and recover high boiling point fraction such as MAA (ammonium salt) and MAM from the column bottom. The recovered bottom fraction can be recycled to the esterification reaction. The low boiling fraction such as ammonia and methanol can be obtained from the column top, and the ammonia gas can be separated from the methanol liquified in the condenser.

Ammonia obtained in the manner described above can be highly purified through absorption process and purification process. This recovered ammonia may be used for the reaction of producing hydrocyanic acid. The hydrocyanic acid may be reacted with acetone in the presence of a catalyst such as small amount of alkali or amine to synthesize ACH, which may be recycled as a starting material. Note that methods for producing hydrocyanic acid using ammonia as a starting material are widely well-known. They include the Andrews process to react the gas mixture of ammonia, methane, and air passed through a catalyst and Sohio process to react propylene, ammonia, and air in the presence of a catalyst in which producing hydrocyanic acid is by-produced during producing acrylonitrile.

Also, methanol after separating ammonia therefrom typically contains the low boiling point fraction such as acetone, and this low boiling point fraction may preferably be separated by distillation. Methanol so obtained is used as a raw material for the above mentioned esterification. Also, this recovered methanol sometimes includes small amount of MMA which is not extracted in the extracting process, and it can be used as a raw material of esterification without any problem.

Generally, the MMA fraction which is obtained from the operation of the extractant recovery column often includes low boiling point fractions such as small amount of acetone, methanol, and methacrylonitrile and high boiling point fractions such as small amount of MMA polymer, and therefore, the MMA fraction is subjected to more distillation operation. To remove these low boiling point and high boiling point fractions can result in highly purified MMA product. Distilling operation for removing both the high and low boiling point fraction may be conducted in any order.

Next, the process for producing MMA according to the present invention is described in detail with reference to Fig. 1 illustrating a specific example thereof. The illustrated is a case where an extractant used has a boiling point higher than that of MMA.

Raw materials for the hydration, ACH 1, water 2, and acetone 3 (in the case of using acetone as the solvent) are continuously supplied to a hydration reactor 4. A HAM production solution 5 is introduced into a thermal decomposition reactor 6 through a filter FT. A mixed gas 7 comprising acetone included in the HAM production solution 5, acetone and hydrocyanic acid produced from decomposition of ACH, and water is separated in the thermal decomposition reactor 6 and is introduced into a distillation column 8. Water is separated from the mixed gas 7 in the distilling column 8, while acetone and hydrocyanic acid are condensed in a condenser 9. A mixed solution of acetone and hydrocyanic acid 10 is recovered. This mixed solution of acetone and hydrocyanic acid 10 is then supplied to the ACH production process and is used for MMA production again. Also, the HAM aqueous solution 11 which is removed the non-reacted ACH and acetone are from the HAM production solution 5 flows out of the thermal decomposition reactor 6 and flows into a cation exchange resin column 12 and an anion exchange resin column 13. The HAM aqueous solution is decationized and deanionized in the ion exchange resin columns 12 and 13, respectively. The HAM aqueous solution 14 is then supplied to a concentration adjusting vessel 15. The concentration adjusting vessel 15 is not always necessary, but owing to concentrating or diluting here, the HAM aqueous solution can keep the constant concentration and be supplied to the next reaction process. The HAM aqueous solution after adjusting concentration 16 is supplied into a dehydration-hydration reactor 17 to proceed dehydration-hydration reaction. A dehydration-hydration product 18 is supplied into an esterification reactor 22 with methanol gas 21, and an MMA product 23 is thus obtained.

Next, the MMA product 23 is supplied to the quench tower 24 and is cooled rapidly. The cooled MMA product is supplied to the extraction column 26 and is contacted with an extractant 25. An extract solution 28 and raffinate solution 27 are then separated from the upper part and the lower part of the column respectively. The raffinate solution 27 is supplied into the methanol recovery column 29 and is distilled. A bottom solution of methanol recovery column 30 containing MAA (ammonium salt) and MAM as a major component can be obtained from the column bottom. This solution 30 may be recycled to the esterification reactor 22. Also, the top gas of methanol recover column 31 which comes from the methanol recovery column 29 can be separated into a methanol solution 33 and an ammonia gas 34 by cooling through the condenser 32. This methanol solution 33 is distilled in the recovery column of low boiling point fraction 35. A low boiling point materials 36 such as acetone is then separated from the column top. Also, methanol 37 is obtained from the bottom of the recovery column of low boiling point fraction 35 and is returned to the esterification reaction as a raw material. Moreover, separated ammonia gas 34 is sent to an ammonia absorption column 38 and an ammonia purification column 39 and a purified ammonia 40 is obtained. This purified ammonia 40 is used as a raw material for acrylonitrile which bring about hydrocyanic acid by-product and for the hydrocyanic acid synthesis. Hydrocyanic acid is a raw material for the ACH synthesis.

Also, the extract solution 28 from the extraction column 26 is supplied to the extractant recovery column 41 and distilled there. The extractant 25 is recovered from the column bottom. This extractant 25 is supplied to the extraction column 26 and is used to extract MMA from MMA product 23 again. Also, the column top solution 42 of the extractant recovery column 41 is distilled in the separation column 43 of low boiling point fraction 42 and a low boiling point materials 44 such as methanol, acetone and methacrylonitrile is separated and removed from the column top. The bottom solution of the separation column of low boiling point fraction 45 is distilled more in the MMA purification column 46. A high boiling point materials 47 such as the extractant and polymers are separated from the column bottom and MMA product 48 is brought about from the column top.

EXAMPLES

The production process according to the present invention is described in detail in conjunction with the following examples. In the following, "%" is considered as the percentage by weight. Also, each component of the reaction solution, the extract solution and the raffinate solution of the extraction, the column top solution, gas and the column bottom solution of the distillation were analyzed by gas chromatography and by liquid chromatography. The concentration of hydrocyanic acid in the reaction solution was analyzed by silver nitrate titration, and ammonia was analyzed by neutralization titration.

Furthermore, examples are described for the following seven processes: "HAM synthesis process", "ACH decomposition process", "HAM purification process", "MMA synthesis process", "MMA extracting process", "methanol recovery process", and "MMA purification process". In addition, "ACH decomposition rate", "MMA extractability" and "Ammonia recovery rate" in the statement are calculated by the equation below.

Equation 1

ACH decomposition rate (%)  =  [(ACH flow rate at the thermal decomposition reactor inlet  -  ACH flow rate at the thermal decomposition reactor outlet)/ACH flow rate at the thermal decomposition reactor inlet] x 100

Equation 2

MMA extractability (%)  =  (Weight of MMA moved to the extract/Weight of MMA in the reaction solution) x 100

Equation 3

Ammonia recovery rate (%)  =  (Weight of recovered Ammonia/Weight of Ammonia in MMA product) x 100

Catalysts Preparation

Preparation of Manganese Dioxide Catalyst (for HAM synthesis)

Sulfuric acid was added to 2 L of aqueous solution of manganese (II) sulfate (concentration: 395 g/liter) to prepare an aqueous solution of manganese (II) sulfate of pH = 1. 557 g of potassium permanganate was added to the solution while keeping the temperature at around 50°C to oxidize the solution, following which 1 L of water was added to this slurry solution. The solution was then aged. The resultant slurry solution was filtered through a suction funnel by an aspirator and dried at 110°C, for 12 hours with a drier to provide 680 g of manganese dioxide. This manganese dioxide was ground into 520 g of powdery catalyst having a particle size of from 16 to 100 mesh.

Preparation of Lanthanum Phosphate Catalyst (for dehydration-hydration of HAM)

212 g of lanthanum oxide ( $La_2O_3$ ) was dissolved in a nitric acid solution, which was heated and concentrated to prepare lanthanum nitrate. Subsequently, water was added to the lanthanum nitrate to provide 2 L of lanthanum nitrate solution. Next, 1 L of aqueous solution containing 203 g of disodium hydrogen phosphate ($Na_2HPO_4$) was added to deposit white precipitation. After stirring the solution at 80°C for 1 hour, white precipitation was rinsed with water sufficiently by decantation and was isolated through a filter and rinsed. The resultant white precipitation was dried at 120°C and was sintered at 400°C for 6 hours in an air stream. The product was then formed into granules having the particle size of from 10 to 16 mesh to prepare a lanthanum phosphate catalyst.

Preparation of $Zr(HPO_4)_2$ catalyst (for esterification reaction)

361 g of zirconium oxychloride ($ZrOCl_2 \bullet 8H_2O$) is dissolved in 500 mL of water. The mixture is added while stirring to the solution in which 1,560 g of sodium dihydrogen phosphate (NaH$_2$PO$_4 \bullet$2H$_2$O) was dissolved in 2 L of 3N-hydrochloric acid and heated at 80°C, to produce white precipitation. After stirring for 1 hour, the white precipitation was rinsed sufficiently by water decantation, isolated and rinsed through a filter.

ACH Production

ACH used for this examples was produced as follows. 580 g of acetone and 10 g of 2%-sodium hydroxide solution were charged into a reactor (a 2-L round-bottom glass flask equipped with a reflux condenser, a stirrer, a thermometer and a liquid inlet), into which 284 g of hydrocyanic acid liquid was introduced while maintaining the temperature at 20°C. After the reaction, sulfuric acid was added to adjust pH to 3.5. Subsequently, non-reacted hydrocyanic acid and acetone were removed under a reduced pressure to obtain 843 g of ACH having the concentration of 99.8%.

Example 1

HAM synthesis process

700 g of the powdery manganese dioxide catalyst obtained in the above-mentioned catalyst preparation and 6300 g of acetone aqueous solution of ACH of 35.4 % (ACH : acetone : water = 1:0.5:7 by molar ratio) were charged into a 10 L suspension reactor (made of SUS; equipped with a stirrer, and a liquid outlet with a metal filter; the catalyst concentration was 10 % in the catalyst suspension solution.) The above acetone aqueous solution of ACH of 35.4 % was supplied continuously with a constant flow pump at a flow rate of 400 g/hr (WHSV: 0.2 g-ACH/g-cat/hr) into it. The content was reacted at 40°C. The resultant HAM production solution was analyzed. The concentrations of the individual components are as follows: HAM 34.0 %, ACH 7.1 %, acetone 12.3 %, hydrocyanic acid 0.1 %, and water 46.5%.

ACH decomposition process

The HAM production solution obtained in the manner mentioned above was supplied into a thermal decomposition reactor with a distillation column (the number of plates is ten) and was decomposed at 100°C, 760 Torr. This resulted in 98.1 % of the decomposition rate of ACH in the HAM production solution. Also, the column top gas of the distillation column was trapped with sodium hydroxide aqueous solution for one hour and was analyzed. The recovery rate of hydrocyanic acid was 96.1 % and the recovery rate of acetone was 97.2 %. 44.3 % HAM aqueous solution was obtained from the column bottom of the thermal decomposition reactor and cooled to 40°C.

HAM purification process

5 L of sulfonic acid type strong acidic cation exchange resin (trade name: Lewatit S100, available from Bayer, Ltd.) and 5 L of quaternary ammonium type strong basic anion exchange resin (trade name: Lewatit M500, available from Bayer, Ltd.) were filled in the respective columns, which were connected to each other in series as a cation exchange resin column and an anion exchange resin column, respectively. The above-mentioned 44.3 % HAM aqueous solution was passed through these columns at the flow rate of SV = 5 hr$^{-1}$. The content of ACH, each cation and anion in the HAM aqueous solution before and after passed through these ion exchange resin column was analyzed. The result is shown in Table 1.

Table 1

| Components | Ion Exchange Resin Column | |
|---|---|---|
| | Inlet Solution | Outlet Solution |
| ACH (%) | 0.2 | trace |
| $Mn^{2+}$ (ppm) | 20 | 0.1 |
| $Na^+$ (ppm) | 5 | 0.2 |
| $SO_4^{2-}$ (ppm) | 200 | 0.1 |

Next, the HAM aqueous solution obtained from above mentioned ion exchange column outlet was concentrated in the 10-L concentration adjusting vessel (made of SUS with an evaporator) to achieve the HAM concentration of 66% (Water/HAM = 3.0 by molar ratio)

MMA synthesis process

The above-mentioned 66% of HAM solution was continuously fed, at 60 g/hr (LHSV = 0.2 g - HAM/g -cat/hr), into a fixed bed dehydration-hydration reactor (made of SUS) filled with 200 ml of lanthanum phosphate catalyst obtained in the above-mentioned catalyst preparation, and was subjected to dehydration-hydration reaction at a temperature of 285°C. Further, both this dehydration-hydration reaction product and vaporized methanol of 85 g/hr (methanol/HAM = 7.0 by molar ratio; LHSV = 0.55 $hr^{-1}$) were continuously fed into a fixed bed esterification reactor filled with 200 ml of $Zr(HPO_4)_2$ catalyst obtained in the above-mentioned catalyst preparation, and were esterified at the temperature of 300°C to produce MMA product. These processes were all continuously carried out. Analysis on the resultant reaction product indicated that it contained 21.4% of MMA, 49.6% of methanol, 4.0 % of ammonia, 18.1 % of water and a small amount of MAA, MAM, acetone, methacrylonitrile.

MMA extraction process

The MMA product was quenched to the temperature of 30 °C. 2000 g/hr of reaction product and 500 g/hr of water as extraction feed, and Shellsol 71 (trade name; available from Shell Japan Ltd. (mixed solvent of saturated aliphatic hydrocarbon having branched chains, which was obtained by distilling at initial boiling point of 179°C and end point of 212°C)) as extractant were continuously fed, at 2000 g/hr (extractant/MMA product ratio = 0.8 by weight), into an extraction column of the theoretical plate number of four, and were contacted in countercurrent for extraction. In this operation, 2025 g/hr of raffinate solution containing 48.5% of methanol and 4.0% of ammonia and 2475 g/hr of extract solution containing 16.4% of MMA were continuously obtained. Further, the extractability of MMA in this operation was 95%.

Further, 0.1% of phenothiazine was added to the above mentioned extract solution as a polymerization inhibitor. The extract solution was fed, at 1200 g/hr, into an extractant recovery column of the theoretical plate number of ten. The content was distilled under the conditions: column top temperature of 45°C, column bottom temperature of 100°C and pressure of 60 Torr. This distillation provided Shellsol 71 solution, at 1016 g/hr, from the bottom of the column, and MMA solution containing 96.2% MMA, at 184 g/hr, from the top of the column. The Shellsol 71 solution is recycled for the former part of the extraction process.

Methanol recovery process

The raffinate solution obtained in the manner described above was fed, at 1200 g/hr, into a methanol recovery column of the theoretical plate number of ten, and was distilled under the conditions: column top temperatures of 45°C, column bottom temperatures of 82°C, and pressure of 340 Torr. Consequently, 550 g/hr of aqueous solution, which contained ammonium salt of MAA and MAM, was separated from the bottom of the column. Further, gas from the top of the column was cooled by a condenser to separate ammonia gas and methanol solution. Separated ammonia was trapped with water, and was analyzed to get 78% recovery of ammonia. Separated methanol solution was further fed into a low boiling point recovery column of the theoretical plate number of ten, and was distilled to remove acetone etc. under the conditions: column top temperature of 58°C, column bottom temperature of 65°C, and pressure of 760 Torr. Methanol having purity of 98% was continuously obtained, at 588 g/hr, from the bottom of the column. The methanol is recycled as the starting material of the esterification.

MMA purification process

The above mentioned column top solution of the extractant recovery column was fed, at 600 g/hr, into a low boiling point recovery column of the theoretical plate number of twenty, and was distilled to remove methanol, methacrylonitrile, and acetone from the top of the column under the conditions: column top temperature of 70°C, column bottom temperature of 80°C, and pressure of 340 Torr. Further, the column bottom solution of the column was fed into a methyl methacrylate purification column of the theoretical plate number of twenty, and was distilled to remove Shellsol 71 and phenothiazine (a polymerization inhibitor) contained in MMA from the bottom of the column under the conditions: column top temperature of 77°C, column bottom temperature of 86°C, and pressure of 340 Torr. In consequence, MMA having purity of 99.8% was obtained, at 561 g/hr, from the top of the column.

Example 2

Example 1 was repeated for the HAM synthesis process, the ACH decomposition process and the former part of the HAM purification process to produce HAM aqueous solution having temperature of 40°C and concentration of 44.3% from the outlet of the ion exchange resin. The concentration of the HAM aqueous solution after HAM purification process was adjusted to be 49.6% (water/HAM = 6.0 by molar ratio) in a concentration adjusting vessel.

MMA synthesis process

The above-mentioned 49.6% of HAM solution was fed, at 40 g/hr (LHSV = 0.2 g•HAM/g-cat/hr), into a fixed bed dehydration-hydration reactor (made of SUS) filled with 100 ml of lanthanum phosphate catalyst obtained in the above-mentioned catalyst preparation, and was continuously subjected to dehydration-hydration reaction at a temperature of 285°C. Further, both this reaction product and vaporized methanol of 55 g/hr (methanol/HAM = 9.0 by molar ratio, LHSV = 0.7 hr$^{-1}$) were continuously fed into a fixed bed esterification reactor filled with 100 ml of Zr(HPO$_4$)$_2$ catalyst obtained in the above-mentioned catalyst preparation, and were continuously esterified at the temperature of 300°C to produce MMA reaction product. Analysis on the resultant reaction product indicated that it contained 16.8% of MMA, 52.5% of methanol, 3.2 % of ammonia, 24.6 % of water and a small amount of MAA, MAM, acetone, methacrylonitrile.

MMA extraction process

The above mentioned MMA product was quenched to the temperature of 30°C.
Subsequently, 2000 g/hr of the MMA product and 500 g/hr of water as extraction feed and n-hexane as extractant were continuously fed, at 2000 g/hr (extractant/MMA product ratio = 1.0 by weight), into an extraction column of the theoretical plate number of four, and were continuously contacted in countercurrent for extraction. In this operation, 1639 g/hr of raffinate solution containing 63.4% of methanol and 3.9% of ammonia, and 2361 g/hr of extract solution containing 13.7% of MMA were continuously obtained. Further, the extractability of MMA in this operation was 96%.
Further, 0.1% of phenothiazine was added to the above mentioned extract solution as a polymerization inhibitor, which was fed, at 1200 g/hr, into an extractant recovery column of the theoretical plate number of twenty. The content was distilled under the conditions: column top temperature of 50°C, column bottom temperature of 80°C, and pressure of 450 Torr. This distillation provided *n*-hexane solution, at 1029 g/hr, from the bottom of the column, and MMA solution containing 88% MMA, at 171 g/hr, from the top of the column. The *n*-hexane solution is recycled for the former part of the extraction process.

Methanol recovery process

The raffinate solution obtained in the operation described above was fed, at 1200 g/hr, into a methanol recovery column of the theoretical plate number of twenty, and was distilled under the conditions: column top temperatures of 50°C, column bottom temperatures of 85°C, and pressure of 450 Torr. Consequently, 390 g/hr of aqueous solution, which contained ammonium salt of MAA and MAM, was separated from the bottom of the column. Further, gas from the top of the column was cooled by a condenser to separate ammonia gas and methanol solution. Separated ammonia was trapped with water, and was analyzed to get 83% recovery of ammonia. Separated methanol solution was further fed into a low boiling point recovery column of the theoretical plate number of ten, and was distilled to remove acetone etc. under the conditions: column top temperature of 49°C, column bottom temperature of 56°C, and pressure of 500 Torr. Methanol having purity of 98% was continuously obtained, at 710 g/hr, from the bottom of the column. The methanol is recycled as the starting material of the above described esterification.

MMA purification process

The above described column top solution of the extractant recovery column was fed, at 600 g/hr, into a low boiling point recovery column of the theoretical plate number of twenty, and was distilled to remove methanol, methacrylonitrile, acetone, and n-hexane from the top of the column under the conditions: column top temperature of 46°C, column bottom temperature of 84°C, and pressure of 350 Torr.

Subsequently, the column bottom solution of the column was fed into a methyl methacrylate purification column of the theoretical plate number of ten, and was distilled to remove phenothiazine etc. from the bottom of the column under the conditions: column top temperature of 55°C, column bottom temperature of 75°C, and pressure of 150 Torr. In consequence, MMA having purity of 99.8% was obtained, at 513 g/hr, from the top of the column.

## Claims

1. A process for producing methyl methacrylate using acetone cyanohydrin, water, and methanol as starting materials, the process comprising the steps of:

    (1) reacting acetone cyanohydrin with water for amidation to produce an $\alpha$-hydroxyisobutyric acid amide production solution;

    (2) thermally decomposing non-reacted acetone cyanohydrin contained in the $\alpha$-hydroxyisobutyric acid amide production solution into a mixture of acetone and hydrocyanic acid and separating the mixture to produce an $\alpha$-hydroxyisobutyric acid amide aqueous solution;

    (3) contacting the $\alpha$-hydroxyisobutyric acid amide aqueous solution with an ion exchange resin to purify the same;

    (4) producing methacrylic acid and/or methacrylic amide through dehydration-hydration of the purified $\alpha$-hydroxyisobutyric acid amide aqueous solution, and adding methanol thereto for esterification to produce methyl methacrylate reaction product and ammonia;

    (5) quenching the methyl methacrylate reaction product with which a hydrophobic organic solvent is contacted for liquid-liquid extraction to provide an extract solution containing methyl methacrylate, and a raffinate solution containing non-reacted methanol and ammonia; distilling the extract solution to separate it into a hydrophobic organic solvent to be recycled as an extractant and methyl methacrylate containing impurities;

    (6) separating, through distillation, methanol and ammonia from the raffinate solution, to recover them separately; and

    (7) purifying, through distillation, methyl methacrylate containing impurities to provide methyl methacrylate as a product.

2. A process for producing methyl methacrylate as claimed in Claim 1, wherein the amount of the acetone serving as a solvent for the reaction in the step (1) is from 0.1 to 2 moles relative to 1 mole of acetone cyanohydrin.

3. A process for producing methyl methacrylate as claimed in Claim 1, wherein the thermal decomposition in the step (2) is operated at a temperature of from 80°C to 100°C and at a pressure of from 100 Torr to 760 Torr.

4. A process for producing methyl methacrylate as claimed in Claim 1, wherein the mixture of acetone and hydrocyanic acid separated in the step (2) is used for a synthesis reaction of acetone cyanohydrin which is a starting material of the step (1).

5. A process for producing methyl methacrylate as claimed in Claim 1, wherein the ion exchange resin in the step (3) is a cation exchange resin and an anion exchange resin.

6. A process for producing methyl methacrylate as claimed in Claim 1, wherein a space velocity obtained by means of dividing a volumetric flow rate per one hour of the α-hydroxyisobutyric acid amide aqueous solution by a volume of the ion exchange resin is from 2 hr$^{-1}$ to 60 hr$^{-1}$ in the step (3).

7. A process for producing methyl methacrylate as claimed in Claim 1, wherein the amount of the water in the dehydration-hydration reaction in the step (4) is from 1 to 15 moles relative to 1 mole of the α-hydroxyisobutyric acid amide.

8. A process for producing methyl methacrylate as claimed in Claim 1, wherein the amount of the methanol in the esterification of the step (4) is from 3 to 15 moles relative to 1 mole of the α-hydroxyisobutyric acid amide.

9. A process for producing methyl methacrylate as claimed in Claim 1, wherein the temperature of cooling the methyl methacrylate reaction product in the step (5) is from 10°C to 70°C.

10. A process for producing methyl methacrylate as claimed in Claim 1, wherein the hydrophobic organic solvent in the step (5) is at least one selected from the group consisting of saturated aliphatic hydrocarbons having a boiling point of from 105°C to 300°C.

11. A process for producing methyl methacrylate as claimed in Claim 10, wherein the hydrophobic organic solvent is at least one selected from the group consisting of saturated aliphatic hydrocarbons having a boiling point of from 120°C to 250°C.

12. A process for producing methyl methacrylate as claimed in Claim 1, wherein the hydrophobic organic solvent in the step (5) is at least one selected from the group consisting of saturated aliphatic hydrocarbons having a boiling point of from 60°C to 95°C.

13. A process for producing methyl methacrylate as claimed in Claim 1, wherein the amount of the hydrophobic organic solvent in the step (5) is from 0.1 to 5 parts by weight relative to 1 part by weight of the methyl methacrylate reaction product.

14. A process for producing methyl methacrylate as claimed in Claim 1, wherein the methanol recovered in the step (6) is used for the esterification in the step (4).

15. A process for producing methyl methacrylate as claimed in Claim 1, wherein the ammonia recovered in the step (6) is used for a reaction to produce hydrocyanic acid.

16. A process for producing methyl methacrylate as claimed in Claim 15, wherein the hydrocyanic acid synthesized from the ammonia recovered in the step (6) is used to produce an acetone cyanohydrin which is a starting material in the step (1).

Fig. 1

**European Patent Office**

# EUROPEAN SEARCH REPORT

Application Number

EP 95 30 3848

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | US-A-4 018 829 (WILHELM GRUBER)<br>* column 2, line 16 - line 28 *<br>* column 3 - column 4; examples 1-3 *<br>* column 4; claims *<br>--- | 1 | C07C69/54<br>C07C67/20<br>C07C67/327<br>C07C67/58<br>C07C231/06<br>C07C231/24 |
| D,A | US-A-4 464 539 (MASAO HASHIMOTO)<br>* column 3, line 6 - line 59 *<br>* column 4, line 15 - line 61 *<br>* column 5, line 19 - line 45 *<br>* column 7 - column 8; examples 2,3 *<br>* column 18 - column 20; claims *<br>--- | 1 | |
| A | FR-A-1 220 771 (UNION CARBIDE CORPORATION)<br>* page 1, left column, paragraph 3 - page 2, left column, paragraph 1 *<br>* page 3, left column, paragraph 3 - paragraph 4 *<br>* page 5 - page 6; examples 8,10 *<br>* page 6; claims *<br>----- | 1 | |

TECHNICAL FIELDS SEARCHED (Int.Cl.6)

C07C

The present search report has been drawn up for all claims

| Place of search | Date of completion of the search | Examiner |
|---|---|---|
| THE HAGUE | 7 September 1995 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)